# EUROPÄISCHE PATENTSCHRIFT

(11) **EP 1 326 588 B1**
(45) Veröffentlichungstag und Bekanntmachung des Hinweises auf die Patenterteilung: **30.08.2023**
(21) Anmeldenummer: 01967355.7
(22) Anmeldetag: 19.09.2001
(51) Int. Cl.: A61K 36/324

(54) **ORALE ZUBEREITUNGSFORMEN VON EXTRAKTEN AUS BOSWELLIA**
ORAL DOSAGE FORMS OF BOSWELLIA EXTRACTS
FORMES DE PREPARATIONS ORALES D'EXTRAITS DE BOSWELLIA

(30) Priorität: 20.09.2000 DE 10058734
(43) Veröffentlichungstag der Anmeldung: 16.07.2003
(60) Teilanmeldung: 19173325.2
(73) Patentinhaber: Catalent Germany Eberbach GmbH, 69412 Eberbach (DE)
(72) Erfinder: FISCHER, Gerhard, 69412 Eberbach (DE); RADDATZ, Klaus, 89561 Dischingen (DE); REIDELSHÖFER, Alfred, 64720 Michelstadt (DE)
(74) Vertreter: dompatent von Kreisler Selting Werner - Partnerschaft von Patent- und Rechtsanwälten mbB
(86) Internationale Anmeldenummer: PCT/EP2001/010821
(87) Internationale Veröffentlichungsnummer: WO 2002/024173

(56) Entgegenhaltungen:
- EP-A- 0 496 705
- EP-A- 0 552 657
- WO-A-96/19212
- WO-A-97/07796
- WO-A-99/20289
- DE-A- 4 444 288
- US-A- 5 744 161
- KULKARNI R R ET AL: "EFFICACY OF AN AYURVEDIC FORMULATION IN RHEUMATOID ARTHRITIS: A DOUBLE-BLIND, PLACEBO-CONTROLLED, CROSS-OVER STUDY" INDIAN JOURNAL OF PHARMACOLOGY, XX, XX, Bd. 24, Nr. 2, 1. Juni 1992 (1992-06-01), Seiten 98-101, XP000566169 ISSN: 0253-7613
- ANONYMOUS: "FDA letter" INTERNET ARTICLE, [Online] 27. März 2001 (2001-03-27), Seiten 1-3, XP002204318 Gefunden im Internet: <URL:http://www.fda.gov/ohrms/dockets/dail ys/01/Jan01/010501/let0422.pdf> [gefunden am 2002-07-01]
- ANONYMOUS: "Ultimate Athlete's pain formula" INTERNET ARTICLE, [Online] 10. Juli 2001 (2001-07-10), Seite 1 XP002204319 Gefunden im Internet: <URL:http://www.biochem-fitness.com/uapf.h tm> [gefunden am 2002-07-01]

## Beschreibung

Gegenstand der vorliegenden Erfindung sind orale Zubereitungsformen von festen zähflüssigen oder flüssigen Boswelliaextrakten oder einer oder mehrerer Boswelliasäuren, deren Derivaten sowie Gemischen derselben.

Weihrauch und Weihrauchzubereitungen werden bereits seit der Antike therapeutisch verwendet. Ende des 19. Jahrhunderts wurde als eine der Hauptkomponenten die kristallisierende Boswelliasäure entdeckt. 1932 isolierten Winterstein und Stein eine Acetyl-Boswelliasäuregemisch, welches aus 85 - 92 % Acetyl-β-Boswelliasäure, 5,0 - 7,0 Gew.-% Acetyl-11-keto-β-Boswelliasäure und geringen Anteilen an Acetyl-α-Boswelliasäure bestand. Dieses Gemisch ist auch heute noch die Basis für die Gewinnung einzelner Boswelliasäuren. Dieses Gemisch wird im Folgenden als Boswelliasäuregemisch nach Winterstein und Stein genannt. Weitere Boswelliasäuren und deren Gemische sind beispielsweise bekannt aus der EP-A-0 755 940 und der JP 42 88 095.

Die WO 99/20289 beschreibt klare, hochkonzentrierte Pflanzenextrakte, die für Weichgelatinekapseln geeignet sind, obwohl die rohen Extrakte ungeeignet sind für eine direkte Verkapselung. Es wird ein Trägermaterial verwendet, in dem der jeweilige Extrakt gelöst wird. Dies wird von allen Pflanzenextrakten behauptet, deren Stammpflanzen therapeutische Verwendungen finden. Alle gängigen Pflanzen werden aufgezählt. Unter diesen Ausgangsmaterialien wird auch Boswellia serrata gum oleoresin erwähnt, d.h. der rohe Weihrauch, der aus ätherischem Öl, Schleim und Gummen und Harz besteht. Ein hochkonzentrierter Extrakt hieraus kann daher je nach Lösungsmittel hochangereicherte Fraktionen von ätherischem Öl, Schleim oder Harzbestandteilen enthalten. Weitere Ausführungen oder Beispiele für Weihrauch oder Extrakte hieraus, sowie deren Bestandteile fehlen.

Aus der DE-A 195 31 067 geht hervor, dass Boswelliasäure oder ihre Derivate zur Hemmung der normalen und gesteigerten Leucocytenelastase und Plasmiumaktivität verwendet werden können. Die WO 96/18407 beschreibt die Verwendung von Weihrauch zur Behandlung der Alzheimer Krankheit. Sowohl in der DE-A 195 31 067 als auch in der WO 96/18407 werden die verschiedensten Applikationsformen aufgezählt und dabei auch orale Applikationsformen wie Tabletten, Dragees, Kapseln, Lösungen, Emulsionen und Sirupe genannt. Im Handel befindet sich ausschließlich ein Präparat in Tablettenform unter der Bezeichnung H 150 (Boswellia serrata). Es enthält 400 mg Weihrauch und verweist auf die traditionelle ayurvedische Medizin.

Die WO 9619212 beschreibt die Verwendung von Boswelliasäuren oder deren Derivaten bei Gehirntumoren. Die EP 552657 beschreibt die Verwendung reiner Boswelliasäuren oder deren Salze für die Phophylaxe und/oder die Bekämpfung von Entzündungsvorgängen, die durch gesteigerte Leukozytenbildung hervorgerufen werden.

Die Erfindung hat sich die Aufgabe gestellt, eine orale Zubereitungsform von Extrakten aus Boswellia in flüssiger Form, in Form einer oder mehrerer Boswelliasäuren oder deren Derivaten sowie Gemischen derselben zur Verfügung zu stellen, welche haltbar, gut einnehmbar, gut bioverfügbar und hoch wirksam ist. Des weiteren sollte ein unangenehmer Geruch und Geschmack vermieden werden. Diese Aufgabe wird dadurch gelöst, dass die Zubereitungsform in Form von Gelatinekapseln vorliegt, wobei es sich um Weichgelatinekapseln handelt.

Es hat sich nämlich überraschenderweise herausgestellt, dass Weichgelatinekapseln gegenüber den handelsüblichen Tabletten oder auch anderen Tabletten eine deutlich höhere Wirksamkeit aufweisen, beispielsweise gegenüber eines Carrageenin-induzierten Rattenpfotenödems, einer Galactosamininduzierten Hepatitis an Mäusen sowie bei der Rinderserumalbumin erzeugten Arthritis an Kaninchen. Dies ist ein deutlicher Hinweis darauf, dass Extrakte aus Boswellia in
flüssiger Form oder in Form einer oder mehrerer Boswelliasäuren in Gelatinekapseln wesentlich besser wirksam sind als in Tabletten. Diese Beobachtung ist durch nichts erklärbar und war sicherlich nicht vorhersehbar.

Gegenstand der vorliegenden Erfindung sind somit die oralen Zubereitungsformen von Boswellia gemäß Patentanspruch 1.

Die Erfindung wird durch die nachfolgenden Beispiele näher erläutert.

### Beispiel 1

Ein Trockenextrakt von Boswellia Serrata mit einem titrimetrisch bestimmten Gehalt von 65 % Gesamt-Boswelliasäuren wurde in Glycerolmonodioleat (Tegomuls O) gelöst und in Weichgelatinekapseln abgefüllt. Die Kapseln enthielten 30 mg Trockenextrakt und 40 mg Propylenglykollaurat. Zum Vergleich wurden Tabletten hergestellt enthaltend 30 mg Trockenextrakt, 27 mg Dicalciumphosphat, 10 mg Lactose, 0,2 mg Aerosil 200 und 0,5 mg Magnesiumstearat.

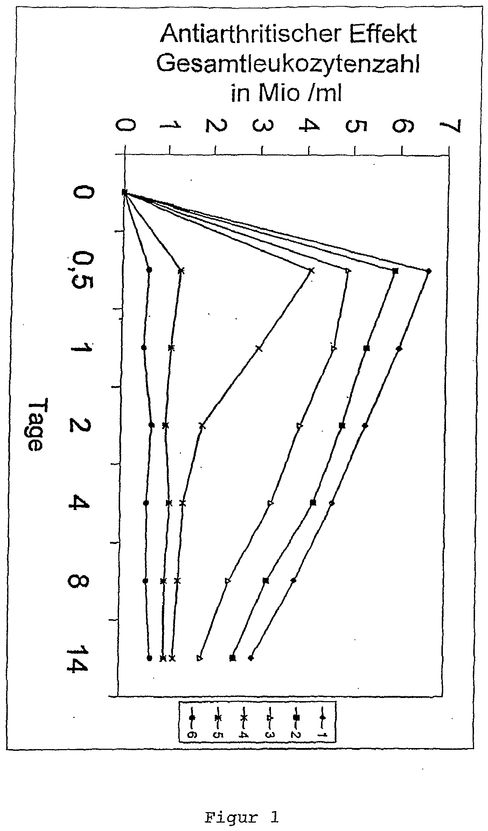

Zubereitungen aus Tabletten und Kapseln wurden Kaninchen über eine Schlundsonde verabreicht. Gemessen wurde die antiarthritische Aktivität einer Rinderserumalbumin erzeugten Arthritis. Diese wurde erzeugt durch Injektion einer sterilen Emulsion aus Rinderserumalbumin und Freund Adjuvants über eine Periode von 14 Tagen. Die oral applizierten Mengen betrugen 25 mg/kg und 100 mg/kg über einen Zeitraum von 14 Tagen. Die Synovialflüssigkeit wurde zu verschiedenen Zeitpunkten gesammelt und jeweils die Gesamtleucocytenzahl gemessen. Die Ergebnisse sind graphisch dargestellt in der Figur 1 und zeigen, dass die Gesamtleucocytenzahl während des Experiments deutlich geringer ist als bei den Vergleichsmedikationen.
Kurve 1 zeigt die Werte der unbehandelten Kontrolle
Kurve 2 zeigt die Werte der Tablette bei einer Menge von 25 mg/kg
Kurve 3 zeigt das erfindungsgemäße Beispiel bei 25 mg/kg
Kurve 4 zeigt das Vergleichsbeispiel bei Applikation von 100 mg/kg
Kurve 5 zeigt das erfindungsgemäße Beispiel bei Applikation von 100 mg/kg Kurve 6 zeigt den Blindwert

### Beispiel 2

Zubereitungen aus Weichgelatinekapseln und Tabletten gemäß Beispiel 1 wurden getestet am Carrageenin-induzierten Rattenpfotenödem. Als Negativ-Kontrolle dienen unbehandelte Tiere; als Positiv-Kontrolle wurden 3 mg/kg Diclofenac-Na gegeben. Die Ergebnisse von je 5 Versuchstieren sind in der nachfolgenden Tabelle zusammengestellt.

| **Medikation** | **Dosis (mg/kg)** | **Carrageeninödem ml** |
|---|---|---|
| Kontrolle | --- | 0,38 |
| Weichgelatinekapsel | 450 | 0,29 |
| Tablette | 450 | 0,43 |

Aus diesen Daten ergibt sich, dass die Tablette deutlich schlechtere Wirkung zeigt, als die Weichgelatinekapsel.

### Beispiel 3

Von einem Trockenextrakt von Boswellia serrata mit einem titrimetrisch bestimmten Gehalt von 65 % Gesamt-Boswelliasäuren und einem per HPLC bestimmten Gehalt von 5 % 11-keto-ß-Boswelliasäure wurden 60 Teile in 70 Teilen Propylenglykollaurat gelöst (Test). Von einer Tablette H 15 (Vergleich) 400 mg Trockenextrakt von Boswellia serrata wurde per HPLC der Gehalt an 11-keto-ß-Boswelliasäure bestimmt. Mit einer gleichgehaltigen Dosis von 11-keto-ß-Boswelliasäure von Test und Vergleich wurde ein Carrageenin-induzierter Rattenpfotenödemtest in verschiedenen Dosierungen durchgeführt. Dabei entsprachen

| Dosierung bezogen auf Boswellia serrata Extrakt (mg/kg) | **Test** (mg/kg) | **Vergleich** (mg/kg) |
|---|---|---|
| 60 | 130 | 119 |
| 180 | 390 | 356 |
| 540 | 1170 | 1067 |

Das Ergebnis ist in der nachfolgenden Tabele dargestellt. Es zeigt, dass nur das Testpräparat in der höchsten Dosis einen Ödem reduzierenden Effekt aufwies.

| **Dosierung (mg/kg)** | **Carrageenan Ödem (ml)** | **% Effekt** |
|---|---|---|
| Kontrolle | 0,38 | - |
| 10723 | | |
| 130 | 0,38 | 0 |
| 390 | 0,50 | 0 |
| 1170 | 0,29 | -23,7 |
| 10724 | | |
| 119 | 0,37 | 0 |
| 356 | 0,39 | 0 |
| 1067 | 0,43 | 0 |
| Diclofenac | | |
| 10 | 0,20 | -47,4 |

### Beispiel 4

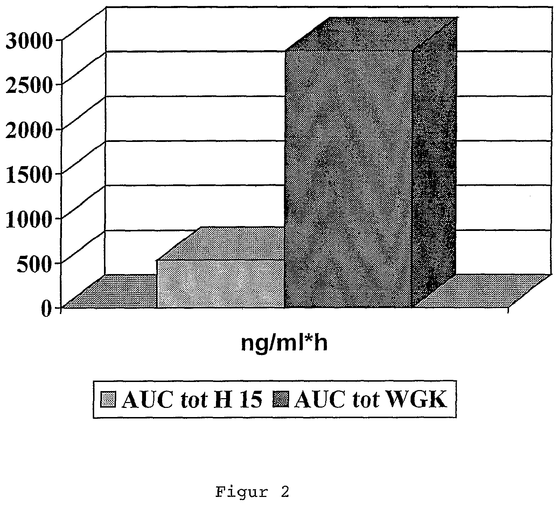

In einer randomisierten, einzeldosierten, Crossover-Bioverfügbarkeitsstudie wurde die Test- und Vergleichsmedikation des Beispiels 3 am Menschen getestet. Um die Vergleichbarkeit zu gewährleisten, wurde wiederum der Gehalt an 11-keto-ß-Boswelliasäure bestimmt. Dies ergab, dass 272 mg Extrakt der Prüfmedikation 400 mg Extrakt des Vergleichspräparats entsprachen. Gegeben wurden jeweils 4 Kapseln (Test) bzw. 4 Tabletten H 15 (Vergleich). Gemessen wurde der Blutspiegel von 11-keto-ß-Boswelliasäure gegen die Zeit. Daraus wurden die Blutspiegelkurven und die pharmakokinetischen Parameter berechnet. Die AUC-Werte sind in der Figur 2 dargestellt.

## Patentansprüche

1. Weichgelatinekapsel enthaltend ausschließlich eine Lösung, welche aus einem Trockenextrakt aus Boswellia, in Form von einer oder mehrerer Boswelliasäuren, deren Derivaten sowie Gemischen derselben, in Propylenglykollaurat besteht.

## Claims

1. A soft gelatin capsule, exclusively containing a solution consisting of a dry extract from *Boswellia* in the form of one or more boswellic acids, derivatives thereof, and mixtures thereof, in propylene glycol laurate.

## Revendications

1. Capsule de gélatine souple, contenant exclusivement une solution, laquelle se compose d'un extrait sec de boswellia, sous la forme d'un ou de plusieurs acides boswelliques, de leurs dérivés ainsi que de mélanges de ceux-ci, dans du laurate de propylène glycol.
